# EUROPÄISCHE PATENTSCHRIFT

(11) **EP 3 960 623 B1**
(45) Veröffentlichungstag und Bekanntmachung des Hinweises auf die Patenterteilung: **12.07.2023**
(21) Anmeldenummer: 20192443.8
(22) Anmeldetag: 24.08.2020
(51) Int. Cl.: B64C 39/02, B64D 11/00, B64F 5/30, A61L 2/10, A61L 9/20

(54) **VORRICHTUNG ZUR DESINFEKTION EINES INNENRAUMS**
DEVICE FOR DISINFECTING AN INTERIOR SPACE
DISPOSITIF DE DÉSINFECTION D'UN ESPACE INTÉRIEUR

(43) Veröffentlichungstag der Anmeldung: 02.03.2022
(73) Patentinhaber: Rister, Frank, 22765 Hamburg (DE); Bartling, Nils, 20457 Hamburg (DE); Kirst, Wulfert, 22765 Hamburg (DE)
(72) Erfinder: BARTLING, Nils, 20457 Hamburg (DE); RISTER, Frank, 22765 Hamburg (DE); WULFERT, Kirst, 22765 Hamburg (DE)
(74) Vertreter: Hauck Patentanwaltspartnerschaft mbB

(56) Entgegenhaltungen:
- EP-A1- 3 354 291
- WO-A1-2018/164845
- CN-U- 207 571 586
- US-A1- 2018 118 337
- US-A1- 2019 216 958

## Beschreibung

Die Erfindung betrifft eine Vorrichtung zur Desinfektion eines Innenraums mit einem Wagen und einer germiziden Lichtquelle. Der Wagen kann beispielsweise durch den Gang einer Flugzeugkabine fahren und die umgebenden Oberflächen, z.B. die Sitze, dem germiziden Licht aussetzen und dadurch eine Desinfektion bewirken.

Die Druckschrift WO 2019/068189 A1 beschreibt die Desinfektion einer Fahrzeugkabine mit einer stationären UV-Beleuchtung.

Die Druckschrift US 2019/0030195 A1 zeigt stationäre UV-Lichtquellen zur Desinfektion einer Flugzeugkabine. Speziell geht es um eine Steuerung der einzelnen Lichtquellen in Abhängigkeit von der Anwesenheit von Personen in bestimmten Zonen der Flugzeugkabine.

Die Druckschrift US 2017/0290935 A1 beschreibt eine Desinfektion von Gängen und Waschräumen in Flugzeugkabinen unter Verwendung von UV-Licht. Die verwendete Vorrichtung umfasst einen Wagen, an dem ein Ausleger mit einer UV-Lichtquelle angeordnet ist.

Die Druckschrift US 2016/0339133 A1 zeigt eine mobile, batteriebetriebene Vorrichtung, die zur Erzeugung von UVC-Licht eine Quecksilber- oder Amalgam-Dampflampe aufweist. Die Verwendung in einem Flugzeugwaschraum wird vorgeschlagen.

Aus den Druckschriften WO 2016/164362 A1 und WO 2016/164364 A1 sind Vorrichtungen zur Desinfektion einer Flugzeugkabine bekannt geworden. Sie umfassen jeweils einen Wagen, der von Hand oder autonom durch einen Gang des Flugzeugs fährt und zu beiden Seiten ausfahrbare Arme aufweist, an denen UVC-Leuchten angeordnet sind. Zur Energieversorgung befindet sich in dem Wagen eine Batterie. Alternativ wird eine Energieversorgung aus einem Stromnetz über ein Kabel angesprochen.

Die Druckschrift WO 2018/164845 A1 zeigt einen ähnlich aufgebauten Wagen zur Desinfektion von Flugzeugkabinen mit UVC-Licht wie die beiden vorstehend diskutierten Druckschriften desselben Anmelders. Der Wagen soll von einer Bedienperson manuell durch einen Gang der Flugzeugkabine geschoben werden und weist einen Schutzschild auf, der die Bedienperson von der UVC-Strahlung abschirmen soll.

Die Druckschrift US 2019/216958 A1 beschreibt eine Vorrichtung zur Verwendung als Drohne zum Desinfizieren eines Raumfahrzeugs.

Die Druckschrift CN 207 571 586 U beschreibt ein unbemanntes Luftfahrzeug zum Sterilisieren.

Die Druckschrift US 2018/118337 A1 zeigt eine Drohne zur Reinigung.

Die Druckschrift EP 3 354 291 A1 offenbart eine mobile Dekontaminationseinheit.

Aus der Firmenbroschüre "Aertos 120-UVC" des Unternehmens Digital Aerolus ist eine ferngesteuerte Kamera-Drohne bekannt geworden, die mit 36 UVC-Leuchtdioden ausgestattet ist. Die maximale Flugzeit soll 10 Minuten betragen.

Davon ausgehend ist es die Aufgabe der Erfindung, eine Vorrichtung zur Desinfektion eines Innenraums zur Verfügung zu stellen, die für einen kommerziellen Einsatz hinreichend robust, effektiv und flexibel einsetzbar ist.

Diese Aufgabe wird gelöst durch die Vorrichtung mit den Merkmalen des Anspruchs 1.Vorteilhafte Ausgestaltungen sind in den Unteransprüchen angegeben.

Die Vorrichtung dient zur Desinfektion eines Innenraums und hat
- einen Wagen, der dazu eingerichtet ist, entlang eines Wegs durch den Innenraum zu fahren,
- eine germizide Lichtquelle und
- eine Drohne, die zur Erzeugung eines Auftriebs mindestens einen elektrischen Antrieb aufweist, wobei
- die germizide Lichtquelle an der Drohne befestigt ist und
- die Drohne zur Energieversorgung der germiziden Lichtquelle und des elektrischen Antriebs über eine elektrische Leitung mit dem Wagen verbunden ist.

Der Innenraum kann beispielsweise ein Hotelzimmer, ein Konferenzraum, ein Theatersaal, ein Fahrgastraum, ein Supermarkt oder ein Verkaufsraum sein. In dem Innenraum gibt es einen Weg, entlang dem der Wagen fahren kann, beispielsweise einen Gang im Supermarkt oder im Reisebus. Andere Bereiche des Innenraums können für den Wagen nicht oder nur schwer zugänglich sein, beispielsweise Bereiche, in denen Regale oder Sitzreihen angeordnet sind. In der Regel gelingt es jedoch in Innenräumen mit unterschiedlichsten Raumaufteilungen, über einen für den Wagen zugänglichen Weg in die Nähe aller zu desinfizierenden Bereiche zu gelangen.

Die germizide Lichtquelle sendet Licht aus, das eine Keime abtötende oder unschädlich machende Wirkung hat. Von dieser Wirkung erfasst werden können Keime aller Art, insbesondere Bakterien, Viren und Pilze. Durch die Bestrahlung mit der germiziden Lichtquelle kann daher idealerweise bei jeglicher Keimbelastung die gewünschte Desinfektion erreicht werden. Bevorzugt wird eine Lichtquelle verwendet, die für Menschen ungefährlich ist, d.h. insbesondere keine karzinogene Wirkung hat. Dies ist jedoch nicht erforderlich, falls beim Einsatz der Vorrichtung eine Exposition von Menschen und gegebenenfalls anderen Lebewesen ausgeschlossen werden kann.

Die Drohne ist ein unbemanntes Luftfahrzeug, das sich in einer Umgebung des Wagens gezielt durch die Luft bewegen kann. Sie weist einen elektrischen Antrieb auf, in der Regel mit einem oder mehreren Rotoren. Flughöhe, Flugrichtung und Fluggeschwindigkeit können von einer geeigneten Steuerung gesteuert werden. Der Aktionsradius der Drohne ist im Wesentlichen durch die elektrische Leitung, mit der die Drohne mit dem Wagen verbunden ist, begrenzt. Die Länge dieser Kabelverbindung kann beispielsweise im Bereich von 1 m bis 20 m liegen, insbesondere im Bereich von 2 m bis 5 m. Innerhalb ihres Aktionsradius kann sich die Drohne gezielt durch den Innenraum bewegen und dabei insbesondere in der Nähe der Drohne befindliche Oberflächen dem von der germiziden, an der Drohne befestigten Lichtquelle abgestrahlten Licht aussetzen.

Die Desinfektionswirkung hängt dabei außer von der Wellenlänge in erster Linie von der Einwirkungsdauer und Intensität des ausgesendeten Lichts ab. Beide Faktoren können durch die Fluggeschwindigkeit der Drohne und den von den zu desinfizierenden Oberflächen eingehaltenen Abstand beeinflusst werden. Zusätzlich zu der Oberflächendesinfektion kann eine Desinfektion der Raumluft bewirkt werden, auch in Verbindung mit Aerosolen. Dies ist besonders vorteilhaft in Verbindung mit kleinen Aerosol-Tröpfchen mit einem Durchmesser von weniger als etwa 3 µm, weil diese nicht mit einem HEPA-Filter aus der Raumluft entfernt werden können.

Eine wichtige Besonderheit der Erfindung besteht in der Kombination eines Wagens mit einer Drohne, deren elektrischer Antrieb und deren Lichtquelle über ein Kabel mit Energie versorgt werden. Diese Kombination bietet den Vorteil, selbst in kompliziert gestalteten, verwinkelten oder beengten Innenräumen praktisch sämtliche relevanten Oberflächen erreichen zu können, und zwar ohne eine aufwändige und potentiell anfällige Mechanik zur Positionierung der Lichtquelle. Es ist auch keine spezielle Anpassung der Vorrichtung an unterschiedliche Innenräume erforderlich, so dass die Vorrichtung besonders flexibel eingesetzt werden kann. Dabei erfolgt die Energieversorgung der Drohne über den Wagen, so dass leistungsstarke Lichtquellen, die für eine schnelle Desinfektion erforderlich sind, verwendet werden können, ohne dass die mögliche Einsatzdauer beschränkt wird.

Es versteht sich, dass die Vorrichtung nicht auf eine einzige, mit dem Wagen verbundene Drohne beschränkt ist. Insbesondere können zwei, drei, vier oder mehr als vier Drohnen eingesetzt werden, die jeweils mit einem Kabel mit dem Wagen verbunden sind und an denen jeweils mindestens eine germizide Lichtquelle befestigt ist. Dadurch können mehrere Oberflächen und/oder unterschiedliche Bereiche derselben Oberfläche gleichzeitig bestrahlt werden. Bei Verwendung mehrerer Drohnen können diese auch mit Kabeln unterschiedlicher Längen mit dem Wagen verbunden sein. Beispielsweise kann eine erste Drohen ein kürzeres Kabel aufweisen, das ihren Einsatz auf einen entsprechend kleinen Umkreis von dem Wagen beschränkt, und eine zweite Drohen kann ein längeres Kabel aufweisen und in größerem Abstand von dem Wagen eingesetzt werden, beispielsweise in einem ringförmigen Umfeld, dass sich nach außen an den von der ersten Drohne abgedeckten Umkreis anschließt.

An dem Wagen können weitere germizide Lichtquellen angeordnet sein, die unabhängig von der Drohne Oberflächen in einer näheren Umgebung des Wagens bestrahlen können. Beispielsweise können in einem unteren Bereich des Wagens eine oder mehrere germizide Lichtquelle angeordnet sein, die auf einen Fußboden gerichtet sind. Alternativ oder zusätzlich können eine oder mehrere germizide Lichtquellen an dem Wagen befestigt sein und ihr Licht in seitlicher Richtung und/oder nach oben abstrahlen. Dadurch kann eine vollständige Desinfektion des Innenraums gegebenenfalls noch schneller erfolgen.

In einer Ausgestaltung ist die Drohne ein Multicopter, d.h. sie weist mehrere, für Auftrieb und Vortrieb zuständige Rotoren auf, insbesondere ein Quadrocopter mit vier Rotoren. Multicopter und vor allem Quadrocopter haben sich wegen ihrer Fähigkeit, auf der Stelle zu schweben und wegen ihrer guten Steuerbarkeit bewährt. Diese Eigenschaften können für eine kabelgebundene Drohne besonders wichtig sein. Sie weisen eine ausreichende Tragfähigkeit auf, so dass auch leistungsstarke und relativ große und/oder schwere Lichtquellen eingesetzt werden können, auch in Verbindung mit relativ langen Kabeln.

In einer Ausgestaltung ist der Innenraum eine Flugzeugkabine und der Weg ein Gang der Flugzeugkabine. Vom Gang der Flugzeugkabine aus sind sämtliche Bereiche der Flugzeugkabine gut zugänglich. Insbesondere sind alle Sitzplätze von einem Gang aus mit wenigen Schritten erreichbar. Gleichzeitig sind die Platzverhältnisse in der Flugzeugkabine beengt und es gibt eine Vielzahl unterschiedlich ausgerichteter Oberflächen, die von den Fluggästen berührt werden. Hierzu zählen die Sitzflächen sowie die Rücken- und Armlehnen der Sitze, aber ebenso die den Fluggästen zugewandten Rückseiten der vor ihnen befindlichen Sitze, Gurte und Gurtschlösser, Bedienelemente für Lüftungsdüsen, Klapptische, Lichtschalter und ein Servicepersonalrufknopf. In dieser Konstellation ist ein Einsatz der erfindungsgemäßen Vorrichtung besonders vorteilhaft. Insbesondere kann die Drohne mit einem relativ kurzen Kabel mit dem Wagen verbunden sein und dennoch alle relevanten Bereiche Flugzeugkabine desinfizieren. Da in der Regel zu beiden Seiten eines Gangs einer Flugzeugkabine Sitzreihen angeordnet sind, ist insbesondere eine Ausgestaltung mit zwei Drohnen sinnvoll. Als Wagen kann insbesondere ein Kabinentrolley eingesetzt werden, insbesondere ein Kabinentrolley mit Luftfahrtzulassung (zum Beispiel ATLAS oder SAE-AS 8056), im *half size-* oder *full size*-Format. In diesem Fall kann der Wagen bzw. die gesamte Vorrichtung an Bord des Flugzeugs transportiert werden. Die Desinfektion der Flugzeugkabine kann dann nach der Landung des Flugzeugs beginnen, insbesondere sobald die Fluggäste die Flugzeugkabine verlassen haben.

In einer Ausgestaltung ist die Lichtquelle eine UVC-Lichtquelle. Die germizide Wirkung von UVC-Licht hat sich in der Praxis bewährt. Mit besonderem Vorteil können Lichtquellen im Fern-UVC-Bereich, d. h. mit einer maximalen Intensität im Wellenlängenbereich von etwa 240 nm bis etwa 100 nm, insbesondere im Bereich von etwa 230 nm bis etwa 200 nm, zum Beispiel bei etwa 222 nm, eingesetzt werden. UVC-Licht in diesem Wellenlängenbereich bietet eine hohe Desinfektionswirkung und ist zugleich unschädlich für größere Lebewesen, weil es nicht so tief in die Haut eindringt, dass das Gewebe geschädigt wird. Insbesondere kann eine karzinogene Wirkung weitgehend ausgeschlossen werden.

In einer Ausgestaltung ist die UVC-Lichtquelle eine Exzimer-Lampe, insbesondere eine Krypton-Chlorid-Exzimer-Lampe. Derartige Lampen weisen eine Wellenlänge von etwa 222 nm auf. Sie werden beispielsweise von der Firma *Far UV Technologies, Inc.* aus Kansas City angeboten. Grundsätzlich können auch beliebige andere UVC-Lichtquellen eingesetzt werden, zum Beispiel Quecksilber-Dampflampen oder UVC-LEDs. Exzimer-Lampen zeichnen sich durch eine hohe Lichtintensität und vorteilhafte Strahlungseigenschaften aus.

In einer Ausgestaltung weist die germizide Lichtquelle zwei röhrenförmige Leuchtmittel auf, die parallel nebeneinander angeordnet sind. Diese Anordnung ist für die Befestigung an einer Drohne besonders günstig und weist zur Desinfektion von Oberflächen eine gute Abstrahlcharakteristik auf.

In einer Ausgestaltung sind an zwei gegenüberliegenden Enden der Anordnung der Leuchtmittel jeweils zwei Rotoren der Drohne angeordnet. Die Drohne ist demnach insbesondere ein Quadrocopter. Das von den vier Rotoren gebildete Rechteck kann von der Leuchtmittel-Anordnung gebildet und/oder von dieser ganz oder teilweise ausgefüllt sein. Auf diese Weise können die Leuchtmittel zur strukturellen Festigkeit der Drohne beitragen, was eine besonders leichte Konstruktion ermöglicht.

In einer Ausgestaltung ist die von den beiden parallel angeordneten, röhrenförmigen Leuchtmitteln definierte Ebene in einem Winkel im Bereich von 5° bis 45° relativ zu einer Rotorebene geneigt angeordnet. Die Rotoren der Drohne rotieren jeweils in einer Ebene, die (zumindest im Schwebeflug) horizontal angeordnet ist. Die gegenüber dieser Horizontalen geneigte Anordnung der Leuchmittelanordnung kann insbesondere für eine nach unten und leicht nach vorn weisende Hauptabstrahlrichtung sorgen, was für eine wirksame Oberflächendesinfektion beispielsweise von Sitzflächen, die im Wesentlichen horizontal ausgerichtet sind, besonders günstig ist.

In einer Ausgestaltung ist in dem Innenraum mindestens eine Sitzreihe angeordnet und die Drohne weist eine elektronische Steuerung auf, die dazu eingerichtet ist, die Drohne automatisch so zu steuern, dass die Drohne sich mit einer vorgegebenen Geschwindigkeit entlang der mindestens einen Sitzreihe bewegt. Zusätzlich kann die Steuerung dazu eingerichtet sein, bei der Bewegung entlang der mindestens einen Sitzreihe einen vorgegebenen Abstand von den Sitzflächen und/oder von den Rückenlehnen der Sitze der Sitzreihe einzuhalten. Hierzu kann die Drohne eine Kamerasystem, das die Anordnung der Sitze erfasst, und/oder eine Abstandsmesseinrichtung aufweisen. Durch eine gezielte Steuerung der Drohne entlang der Sitzreihe wird sichergestellt, dass alle Sitze der Sitzreihe mit einer für die angestrebte Desinfektion ausreichenden Dosis des germiziden Lichts bestrahlt werden. Die Sitzreihe kann beispielsweise in einem Kino- oder Theatersaal oder in einer Flugzeugkabine angeordnet sein.

In einer Ausgestaltung weist der Wagen einen ausfahrbaren Mast auf, der in einer eingefahrenen Position im Inneren des Wagens aufgenommen ist und in einer ausgefahrenen Position nach oben aus dem Wagen herausragt, wobei die elektrische Leitung mit dem Mast verbunden ist. Für einen störungsfreien Flugbetrieb der Drohne kann es von Vorteil sein, wenn die elektrische Leitung in relativ großer Höhe an dem Wagen befestigt ist, zum Beispiel in einer Höhe im Bereich von 1,20 m bis 1,80 m und damit zum Beispiel oberhalb der Kopfstützen typischer Sitze. Hierzu dient der Mast, wobei die elektrische Leitung bevorzugt an einem oberen Ende des Mastes angeschlagen und/oder aus dem Mast herausgeführt ist. Ein besonderer Vorteil des ausfahrbaren Masts ist, dass der Mast in seiner eingefahrenen Position vollständig im Inneren des Wagens aufgenommen ist, sodass der Wagen platzsparend verstaut werden kann. Dies ist beispielsweise wichtig, wenn die Vorrichtung an Bord eines Flugzeugs mitgeführt werden soll.

In einer Ausgestaltung weist die Vorrichtung einen elektrischen Fahrantrieb und eine elektronische Steuerung für den Wagen auf, wobei die Steuerung dazu eingerichtet ist, den Wagen autonom entlang des Wegs zu steuern. Grundsätzlich kann die Vorrichtung auch mit einem Wagen ohne eigenen Fahrantrieb eingesetzt werden, beispielsweise wenn der Wagen von einer Bedienperson entlang des Wegs geschoben oder gezogen wird. Mit einem eigenen Fahrantrieb für den Wagen ist hierfür keine Bedienperson erforderlich. Dies gilt in besonderer Weise in Verbindung mit der autonomen Steuerung für den Wagen. Anders als beispielsweise bei einer Fernsteuerung für den Wagen, die ebenfalls möglich ist, ist dann auch keine Bedienperson für die Steuerung des Wagens erforderlich. Damit der Wagen autonom seinen Weg findet, kann er mit einem geeigneten Navigationssystem ausgestattet sein, insbesondere mit einem Kamerasystem. Das Kamerasystem kann eine Umgebung des Wagens erfassen und den Weg selbsttätig erkennen. Alternativ oder zusätzlich kann der abzufahrende Weg eine Markierung aufweisen, beispielsweise optisch erkennbare Wegmarken, Induktionskabel oder magnetische Markierungen. Die autonome Steuerung des Wagens kann bei der Fahrt entlang des Wegs insbesondere die Fahrgeschwindigkeit oder geeignete Zwischenstopps so wählen, dass der Drohne ausreichend Zeit für die Desinfektion der angrenzenden Bereiche, beispielsweise der genannten Sitzreihen, zur Verfügung steht.

In einer Ausgestaltung umfasst die Vorrichtung ein Stromkabel, das mit dem Wagen verbunden ist und einen Stecker zum Anschließen an ein Versorgungsnetz, insbesondere an ein Flugzeugbordnetz, aufweist. Grundsätzlich kann der Wagen auf beliebige Art und Weise mit elektrischer Energie für die Drohne und gegebenenfalls für weitere Elemente wie einen eigenen elektrischen Fahrantrieb, eine Steuerung usw. aufweisen. Beispielsweise kann eine entsprechend dimensionierte, wiederaufladbare Batterie in den Wagen integriert werden. Für viele Einsatzzwecke, insbesondere für einen Einsatz an Bord eines Flugzeugs, ist eine Batterieversorgung jedoch problematisch, weil größere Batterien nicht ohne weiteres an Bord eines Flugzeugs mitgeführt werden dürfen. Weitere Schwierigkeiten können sich hinsichtlich des Aufladens einer solchen Batterie ergeben. Die Versorgung des Wagens über ein Stromkabel aus einem Versorgungsnetz ist eine praktikable Alternative, mit der insbesondere auch leistungsstarke germizide Lichtquellen und elektrische Antriebe für die Drohne einfach und zuverlässig versorgt werden können. Nach dem Verstauen des Stromkabels ergeben sich auch keine besonderen Risiken hinsichtlich des Transports an Bord eines Flugzeugs. Das Versorgungsnetz kann ein in dem betreffenden Innenraum verfügbares Hausnetz (zum Beispiel 230 V Wechselstrom) oder ein anderes geeignetes Versorgungsnetz sein. Das Flugzeugbordnetz kann beispielsweise eine Nennspannung von 28 V DC aufweisen oder eine Nennspannung von 115 V AC oder 230 V AC aufweisen bei einer Frequenz von 400 Hz oder 800 Hz.

In einer Ausgestaltung weist die Vorrichtung eine Aufrollvorrichtung für das Stromkabel auf, die dazu ausgebildet ist, den Wagen durch Aufrollen des Stromkabels entlang eines vorgegebenen Fahrwegs zu ziehen. In bestimmten Anwendungsfällen kann der Wagen dadurch besonders einfach fortbewegt werden. Dies gilt zum Beispiel in einer Flugzeugkabine, die ein sogenanntes *In-cabin-belt-System* aufweist.

In einer Ausgestaltung weist die Vorrichtung eine Aufrollvorrichtung für das Stromkabel auf, wobei die elektronische Steuerung für den Wagen dazu ausgebildet ist, das Stromkabel während der Fahrt automatisch in Abhängigkeit von einer Fahrgeschwindigkeit und/oder Fahrtrichtung auf- und abzurollen. Durch ein solches, automatisches Auf- und Abrollen des Stromkabels kann der Wagen gegebenenfalls auch komplizierte und/oder längere Wege abfahren, ohne dass das Stromkabel besondere Schwierigkeiten bereitet. Zum Beispiel kann der Wagen entlang eines Mittelgangs durch eine Flugzeugkabine fahren, beispielsweise beginnend ganz vorn beim Cockpit, wo der Stecker an das Flugzeugbordnetz angeschlossen ist. In diesem Fall wird das Stromkabel auf dem Weg zum hinteren Ende der Flugzeugkabine abgerollt und auf dem Rückweg aufgerollt. Dies kann vollautomatisch geschehen, sodass das Kabinenpersonal die Vorrichtung lediglich in der Nähe des Cockpits in Betrieb nehmen und nach abgeschlossener Desinfektion dort wieder verstauen muss.

In einer Ausgestaltung weist die Vorrichtung ein Fahrantriebsmodul auf, auf dem der Wagen angeordnet und befestigt werden kann, wobei das Fahrantriebsmodul insbesondere ein Raupenfahrwerk aufweist. In dieser speziellen Kombination weist der Wagen einerseits Rollen oder Räder auf, auf denen er von Hand geschoben oder gezogen werden kann, beispielsweise um den Wagen an Bord eine Flugzeugkabine zum Beispiel in einer Aufnahme für einen Kabinentrolley zu verstauen. Zusätzlich gibt es ein Fahrantriebsmodul, auf dem der Wagen entlang des Wegs gefahren werden kann. Das Fahrantriebsmodul kann hierzu beispielsweise mehrere, teilweise gelenkte Räder aufweisen. Insbesondere kann es sich um ein Raupenfahrwerk handeln, welches bei den relativ geringen Fahrgeschwindigkeiten einen besonders störungsfreien Betrieb ermöglicht, da es zum Beispiel kleinere Hindernisse besonders einfach überwinden kann.

In einer Ausgestaltung weist das Fahrantriebsmodul eine Rampe auf, über die der Wagen von Hand auf das Fahrantriebsmodul gefahren werden kann. Hierzu kann das Fahrantriebsmodul auf dem Boden platziert werden. Anschließend wird der Wagen von Hand über die Rampe auf das Fahrantriebsmodul gefahren und in der gewünschten Position auf dem Fahrantriebsmodul fixiert. Für die weitere Fahrt werden die Räder des Wagens dann nicht mehr benötigt.

In einer Ausgestaltung weist der Wagen eine Aufnahmefach für die Drohne und/oder ein Aufnahmefach für das Fahrantriebsmodul auf, sodass die Drohne einschließlich der elektrischen Versorgungsleitung bzw. das Fahrantriebsmodul im Inneren des Wagens verstaut werden kann. Dies ist beispielsweise an Bord eines Flugzeugs besonders wichtig, um den Wagen in einer standardisierte Aufnahme verstauen zu können. Zugleich sind die Drohne und/oder das Fahrantriebsmodul während des Transports der Vorrichtung optimal geschützt.

Nachfolgend wird die Erfindung anhand von in Figuren gezeigten Ausführungsbeispielen näher erläutert. Es zeigen:
- Fig. 1:: eine Vorrichtung zur Desinfektion eines Innenraums, deren Elemente im Inneren ihres Wagens verstaut sind,
- Fign. 2 bis 7:: die Vorrichtung aus Fig. 1 in unterschiedlichen Aufbauzuständen,
- Fig. 8:: die Vorrichtung aus Fig. 1 während der Desinfektion von Sitzreihen eine Flugzeugkabine,
- Fig. 9:: eine Drohne der Vorrichtung aus Fig. 1 in einer Ansicht von oben,
- Fig. 10:: die Drohne aus Fig. 9 in einer Ansicht von der Seite,
- Fig. 11:: die Drohne aus Fig. 9 in einer perspektivischen Darstellung,
- Fig. 12:: eine weitere Vorrichtung zu Desinfektion eines Innenraums während der Desinfektion von Sitzreihen eine Flugzeugkabine.

Alle Figuren sind schematisch und beziehen sich beispielhaft auf eine Anwendung der Vorrichtung in einer Flugzeugkabine.

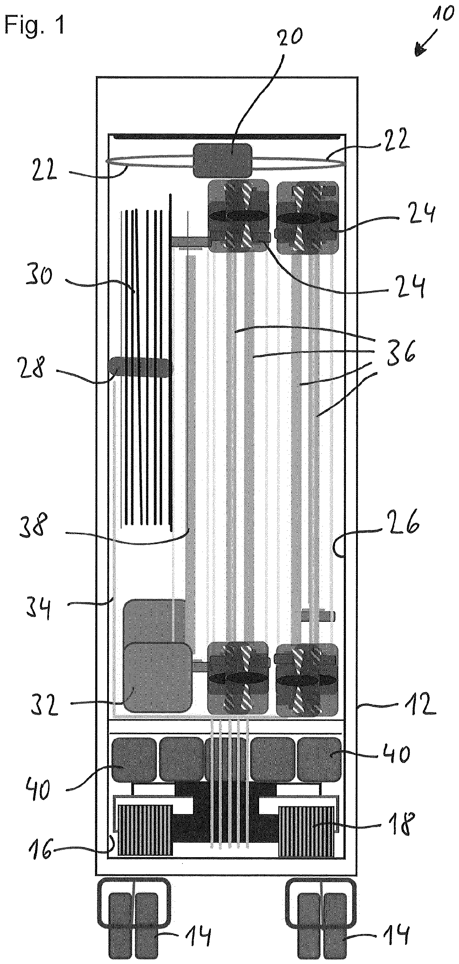

Figur 1 zeigt eine Vorrichtung 10 zur Desinfektion einer Flugzeugkabine, die als Wagen einen luftfahrtzugelassenen Kabinentrolley 12 mit einem quaderförmigen, aufrecht stehenden Korpus und vier an dessen Unterseite angeordneten Rädern 14 aufweist. Derartige Kabinentrolleys 12 werden häufig zur Versorgung der Fluggäste mit Snacks und Getränken eingesetzt und zu diesem Zweck von Hand durch einen Gang der Flugzeugkabine geschoben oder gezogen. In dem in der Fig. 1 gezeigten Zustand der Vorrichtung 10 befinden sich sämtliche Elemente, die für die Desinfektion benötigt werden, innerhalb des Korpus, sodass die gesamte Vorrichtung 10 wie ein gewöhnlicher Kabinentrolley 12 in einem hierfür vorgesehenen Stauraum an Bord des Flugzeugs untergebracht werden kann.

In einem unteren Bereich des Korpus des Kabinentrolleys 12 befindet sich ein Aufnahmefach 16, in dem ein Fahrantriebsmodul 18 untergebracht ist. Hierzu ist das Aufnahmefach 16 so angepasst, dass das Fahrantriebsmodul 18 darin sicher gehalten ist, beispielsweise durch passgenaue Ausführung und/oder eine spezielle Befestigungseinrichtung für das Fahrantriebsmodul 18.

In einem oberen Bereich des Korpus des Kabinentrolleys 12 befindet sich ein nur andeutungsweise erkennbarer, nach oben ausfahrbarer Mast 20, von dem zwei in der Fig. 1 nur teilweise sichtbare, elektrische Leitungen 22 ausgehen. Diese beiden elektrischen Leitungen 22 führen zu jeweils einer Drohne 24. Die beiden Drohnen 24 sind in einem weiteren Aufnahmefach 26 im Korpus des Kabinentrolleys 12 untergebracht.

Auch das weitere Aufnahmefach 26 ist so angepasst, dass die Drohnen 24 darin sicher gehalten sind, beispielsweise durch passgenaue Ausführung und/oder eine spezielle Befestigungseinrichtung die beiden Drohnen 24.

Seitlich des weiteren Aufnahmefachs 26 befindet sich eine Aufrollvorrichtung 28, auf der ein Stromkabel 30 aufgerollt ist. Über das Stromkabel 30, das an seinem nicht gezeigten, freien Ende einen Stecker aufweist, kann die Vorrichtung 10 an ein Flugzeugbordnetz angeschlossen werden.

Weiterhin weist die Vorrichtung 10 im Inneren des Kabinentrolleys 12 zwei Transformatoren 32 auf, über die die einzelnen Elemente der Vorrichtung 10 mit elektrischer Energie aus dem Flugzeugbordnetz versorgt werden. Diesbezüglich sind in der Fig. 1 andeutungsweise weitere elektrische Leitungen 34 gezeigt, die von der Aufrollvorrichtung 28 für das Stromkabel 30 zu den Transformatoren 32 bzw. von dort zu dem Fahrantriebsmodul 18 führen.

Wie Fig. 1 weiter zeigt, weist die Vorrichtung 10 eine Anzahl germizider Lichtquellen auf: Zu jeder der beiden Drohnen 24 gehören zwei germizide Lichtquellen 36. Eine weitere germizide Lichtquelle 38 ist dem ausfahrbaren Mast 20 zugeordnet. Noch fünf weitere germizide Lichtquellen 40 sind dem Fahrantriebsmodul 18 zugeordnet. Sämtliche germiziden Lichtquellen 36, 38, 40 sind im Inneren des Kabinentrolleys 12 sicher verstaut. Bei der beispielhaft gezeigten Vorrichtung 10 weist jede der germiziden Lichtquellen 36, 38, 40 ein eine Krypton-Chlorid-Exzimer-Lampe auf, die UVC-Licht mit einer Wellenlänge von etwa 222 nm aussendet.

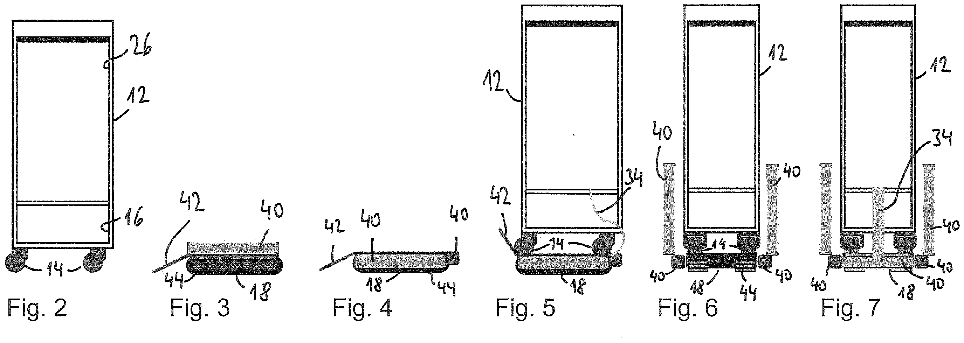

Weitere Einzelheiten der Vorrichtung 10, insbesondere wie diese von dem in Fig. 1 gezeigten Zustand, in dem sämtliche Elemente innerhalb des Kabinentrolleys 12 verstaut sind, in einen Betriebszustand überführt werden kann, werden anhand der Fign. 2 bis 7 erläutert. Fig. 2 zeigt den Kabinetttrolley 12 in einer Ansicht von der Seite, wobei die im Inneren des Wagens angeordneten Elemente nicht dargestellt sind. Deutlich erkennbar sind jedoch die beiden Aufnahmefächer 16 und 26.

Figur 3 zeigt das Fahrantriebsmodul 18, das gemeinsam mit den fünf weiteren germiziden Lichtquellen 40 dem Aufnahmefach 16 entnommen und auf den Boden gestellt wurde. Gut erkennbar ist eine Rampe 42 des Fahrantriebsmoduls 18, die bereits heruntergeklappt wurde. Das Fahrantriebsmodul 18 weist ein Raupenfahrwerk mit zwei seitlich nebeneinander angeordneten Raupen 44 auf.

Figur 4 zeigt, dass im nächsten Aufbauschritt drei der fünf weiteren germiziden Lichtquellen 40 in ihrer jeweils vorgesehenen Position an dem Fahrantriebsmodul 18 angeordnet worden sind. Dies geschieht durch eine geeignete Klappvorrichtung oder beispielsweise durch einfaches Einstecken der weiteren germiziden Lichtquellen 40 in dafür vorgesehene Aufnahmen am Fahrantriebsmodul 18. Wie die Fig. 4 zeigt, befindet sich eine weitere germizide Lichtquelle 40 dann in geringer Höhe über dem Boden an einem vorderen Ende des Fahrantriebsmoduls 18, sodass sie während der Fahrt insbesondere den unterhalb und vor dem Fahrantriebsmodul 18 befindlichen Boden bestrahlt. Zwei der weiteren germiziden Lichtquellen 40 sind seitlich an dem Fahrantriebsmodul 18 angeordnet worden, ebenfalls in geringer Höhe über dem Boden. Sie bestrahlen insbesondere Fußbodenbereiche seitlich des Wegs, entlang dem die Vorrichtung 10 gefahren wird.

Nachdem das Fahrantriebsmodul 18 in die in Fig. 4 gezeigte Stellung gebracht worden ist, kann der Kabinentrolley 12 über die Rampe 42 einfach auf das Fahrantriebsmodul 18 gefahren werden. Dann ergibt sich die Fig. 5 gezeigte Anordnung, bei der sich der Kabinentrolley 12 mit seinen Rädern 14 auf dem Fahrantriebsmodul 18 befindet. In dieser Position wird der Kabinentrolley 12 auf dem Fahrantriebsmodul 18 fixiert. Wie in Fig. 5 angedeutet, kann dies teilweise durch Hochklappen der Rampe 42 geschehen. Im einfachsten Fall kann der Kabinentrolley 12 mit seinen Rädern 14 in passgenau ausgearbeitete Vertiefungen an der Oberseite des Fahrantriebsmoduls 18 eingesetzt werden. Alternativ oder zusätzlich ist eine Fixierung des Kabinentolleys 12 auf dem Fahrantriebsmodul 18 beispielsweise mit Riemen oder Klemmhebeln möglich.

Figur 5 zeigt außerdem, dass das Fahrantriebsmodul 18 über eine der weiteren Leitungen 34 mit dem Kabinentrolley 12 bzw. den darin angeordneten Transformatoren 32 in Verbindung steht und somit über das Stromkabel 30 mit elektrischer Energie versorgt werden kann.

In der Figur 6 ist die Anordnung aus Fig. 5 in einer Ansicht von hinten gezeigt, wobei zusätzlich zu den drei bereits beschriebenen, weiteren germiziden Lichtquellen 40 zwei weitere germizide Lichtquellen 40 in aufrechtstehender Anordnung seitlich eines unteren Abschnitts des Kabinentolleys 12 befestigt worden sind. In dieser Stellung bestrahlen sie beim Fahren der Vorrichtung 10 entlang eines Gangs einer Flugzeugkabine insbesondere die dem Gang zugewandten, seitlichen Flächen der angrenzenden Sitze.

Figur 7 zeigt die Anordnung aus Fig. 6 in einer Ansicht von vom, bei der der Blick auf die Vorderseite des Fahrantriebsmoduls 18 mit der dort quer befestigten, weiteren germiziden Lichtquelle 40 gerichtet ist. Ebenfalls erkennbar ist die weitere elektrische Leitung 34, die das Fahrantriebsmodul 18 an den Kabinentolley 12 anschließt.

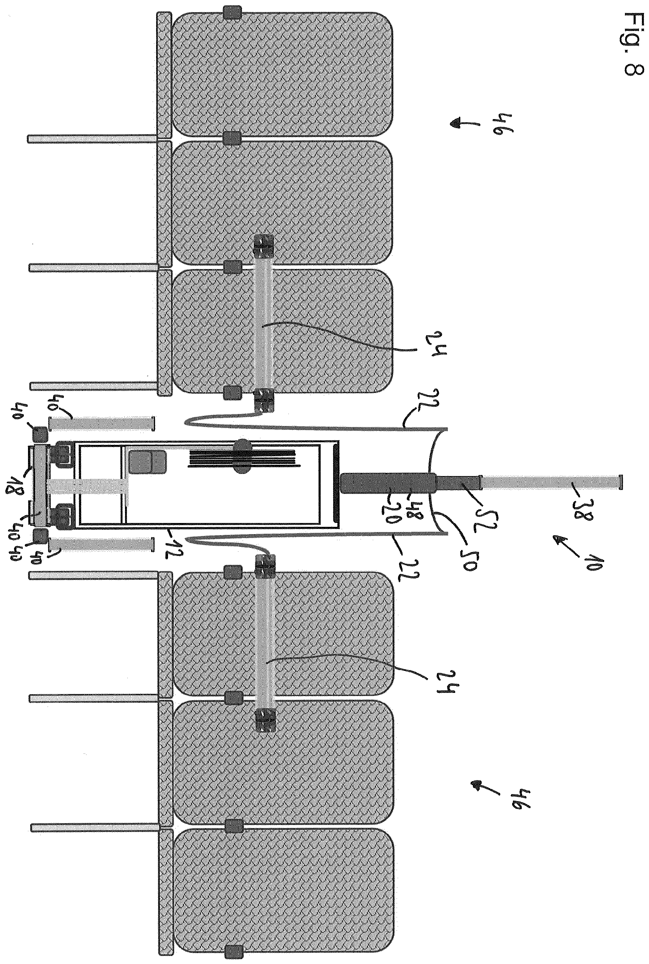

In Figur 8 ist die Vorrichtung 10 schließlich im Einsatz zu sehen, während der Desinfektion zweier zu beiden Seiten eines Gangs angeordneten Sitzreihen 46 in einer Flugzeugkabine. Der untere Teil der Vorrichtung 10 befindet sich in dem zur Fig. 7 erläuterten Zustand, mit dem Kabinentrolley 12 auf dem Fahrantriebsmodul 18 und der beschriebenen Anordnung der weiteren, germiziden Lichtquellen 40. Im Unterschied zur Fig. 7 befinden sich nun die beiden Drohnen 24 zu beiden Seiten des Kabinentrolleys 12 im Schwebeflug. Außerdem wurde der ausfahrbare Mast 20 ausgefahren, sodass er nach oben aus dem Kabinentrolley 12 herausragt.

Man erkennt in Fig. 8, dass der Mast 20 ein Teleskopmast ist, wobei die beiden elektrischen Leitungen 22 am oberen Ende eines äußeren Teleskopmastabschnitts 48 aus dem Mast 20 herausgeführt sind. Ausgehend von kleinen, etwa horizontal angeordneten Abstandshaltern 50, die seitlich aus dem Teleskopmastabschnitt 48 hervorstehen, hängen die elektrischen Leitungen 22 frei nach unten und führen unter Ausbildung einer Schlaufe jeweils zu einer der beiden Drohnen 24.

Ein innerer Teleskopmastabschnitt 52 des ausfahrbaren Masts 20 ragt nach oben aus dem äußeren Teleskopmastbschnitt 48 heraus und trägt an seinem freien Ende die weitere germizide Lichtquelle 38, die senkrecht nach oben weist. In dieser Stellung dient die weitere germizide Lichtquelle 38 insbesondere zur Desinfektion von in Fig. 8 nicht dargestellten Gepäckfächem, die sich im oberen Bereich der Flugzeugkabine zu beiden Seiten des Gangs befinden.

Mithilfe einer nicht gezeigten Steuerung können die beiden Drohnen 24 jeweils in einem vorgegebenen Abstand gezielt entlang der Sitzreihen 46 bewegt werden, sodass sie nacheinander jede der Sitzflächen und jede der Rückenlehnen der Sitze der Sitzreihen 46 bestrahlt, bis die gewünschte Desinfektionswirkung erreicht ist. Hierzu führt die Steuerung die Flugbewegung mit einer geeigneten, vorgebbaren Fluggeschwindigkeit und einem geeigneten, vorgegebenen Abstand von den zu bestrahlenden Oberflächen aus. Man erkennt in Fig. 8, dass die Abmessungen der Drohnen 24, insbesondere die Länge der verwendeten germiziden Lichtquellen 36, an die Breite der einzelnen Sitze angepasst sind: Die Leuchtmittel haben eine Länge, die gleich groß oder etwas größer ist, als eine Breite der Sitze. Dadurch können während eines geradlinigen Flugs der Drohne 24 jeweils eine Sitzfläche und eine Rückenlehne über ihre gesamte Breite desinfiziert werden.

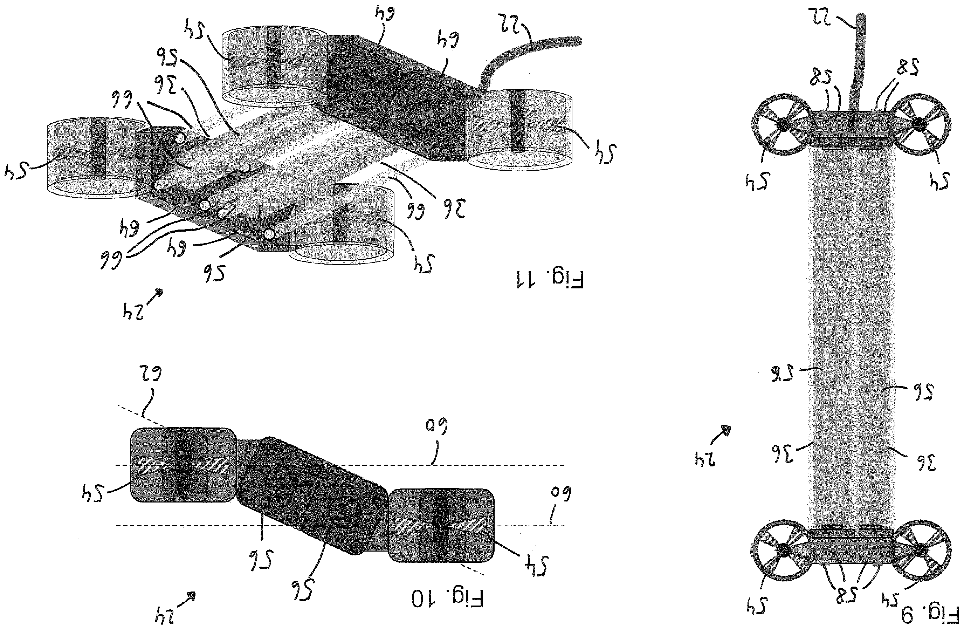

Weitere Einzelheiten der Drohnen 24 sind besser in den Fign. 9 bis 11 erkennbar. In der Fig. 9, einer Ansicht von oben, erkennt man, dass es sich bei den Drohnen 24 um Quadrocopter mit vier Rotoren 54 handelt. Die beiden germiziden Lichtquellen 36 weisen röhrenförmige Leuchtmittel 56 auf, die parallel nebeneinander angeordnet sind. An den beiden gegenüberliegenden Enden dieser Anordnung der Leuchtmittel 56 sind jeweils zwei der Rotoren 54 der Drohne 24 angeordnet. Ebenfalls erkennbar sind mehrere Sensoren 58, mit denen zwecks Steuerung der Drohne 24 während des Flugs eine Umgebung dr Drohne 24 erfasst werden kann. Die elektrische Leitung 22 ist an einem Ende der Drohne 24 zwischen zwei Rotoren 54 befestigt. Sie versorgt sowohl elektrische Antriebe der Rotoren 54 als auch die germiziden Lichtquellen 36 mit elektrischer Energie.

Figur 10 zeigt die Drohne 24 aus Fig. 9 in einer Ansicht von der Seite. Man erkennt, dass die Rotoren 54 jeweils eine Rotorebene 60 aufweisen, die im Schwebeflug etwa horizontal ausgerichtet ist. Die beiden parallel angeordneten, röhrenförmigen Leuchtmittel 56 definieren eine weitere Ebene 62, die in einem Winkel von etwa 15° relativ zu den Rotorebenen 60 geneigt angeordnet ist. Die beiden Rotorebenen 60 sind entsprechend in vertikaler Richtung versetzt angeordnet.

Figur 11 zeigt eine perspektivische Ansicht, die den erläuterten Aufbau der Drohnen 24 nochmals verdeutlicht. Man erkennt, dass die germiziden Lichtquellen 36 jeweils entlang ihrer Längsachse eines der Leuchtmittel 56 aufweisen und darum herum eine Tragstruktur, die im gezeigten Beispiel zwei rechteckige Endplatten 64 und vier diese miteinander verbindende Stäbe 66 aufweist. Durch diese Tragstruktur ist das Leuchtmittel 56 vor Beschädigungen geschützt. Zugleich bilden die germiziden Lichtquellen 36 ein wesentliches Element einer Tragstruktur der Drohne 24.

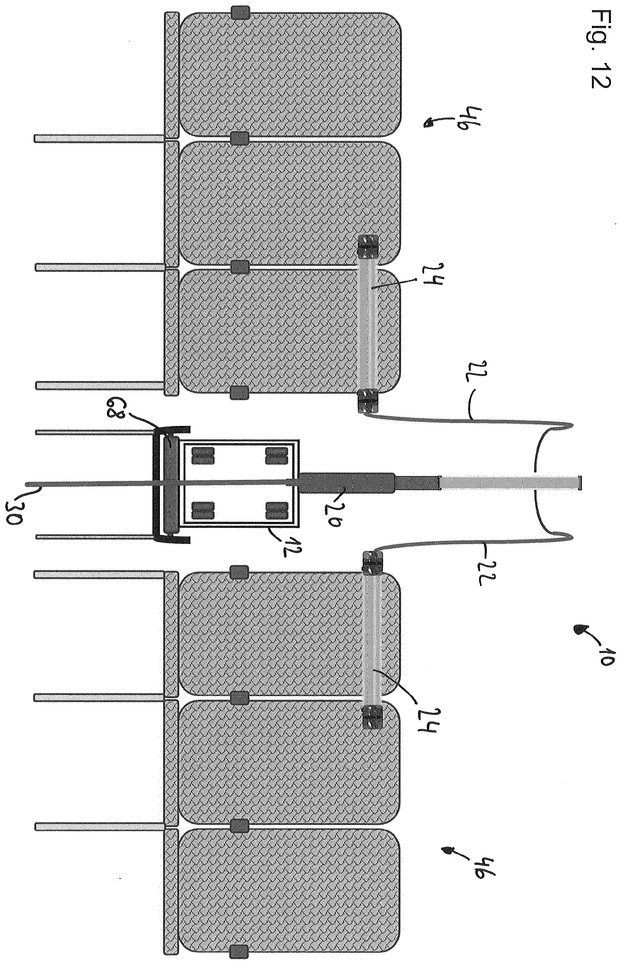

Figur 12 zeigt eine andere Vorrichtung 10 zur Desinfektion einer Flugzeugkabine, die ebenfalls einen Kabinentrolley 12 als Wagen und zwei Drohnen 24 aufweist. Für die Bewegung des Kabinentrolleys 12 entlang des Gangs sorgt jedoch kein Fahrantriebsmodul 18 wie bei dem zuvor beschriebenen Ausführungsbeispiel, sondern der Kabinenrolley 12 wird auf einer Rückseite liegend auf einem entlang des Gangs verlaufenden Transportband 68 angeordnet. Das Transportband 68 ist Teil eines sogenannten *In-cabin-belt-Systems,* mit dem Gepäck entlang des Gangs transportiert werden kann. Im Zusammenhang mit der erfindungsgemäßen Vorrichtung wird die Aufrollvorrichtung 28 und das damit aufgerollte Stromkabel 30 des Kabinentrolleys 12 dazu verwendet, die Vorrichtung 10 entlang des Transportbands 68 zu ziehen. Die Anbindung der Drohnen 24 über elektrische Leitungen 22 erfolgt wie im vorigen Ausführungsbeispiel über einen ausfahrbaren Mast 20, der jedoch so an dem Kabinentrolley 12 angeordnet ist, dass er aus einer in liegender Position oben befindlichen Seite aus dem Kabinentrolley 12 herausragt.

### Liste der Bezugszeichen:

- 10: Vorrichtung zur Desinfektion eines Innenraums/einer Flugzeugkabine
- 12: Kabinentrolley
- 14: Räder
- 16: Aufnahmefach für Fahrantriebsmodul
- 18: Fahrantriebsmodul
- 20: ausfahrbarer Mast
- 22: elektrische Leitung
- 24: Drohne
- 26: Aufnahmefach für eine Drohne
- 28: Aufrollvorrichtung
- 30: Stromkabel
- 32: Transformator
- 34: weitere elektrische Leitung
- 36: germizide Lichtquelle (Drohne)
- 38: weitere germizide Lichtquelle (Mast)
- 40: weitere germizide Lichtquelle (Fahrantriebsmodul)
- 42: Rampe
- 44: Raupe
- 46: Sitzreihe
- 48: äußerer Teleskopmastabschnitt
- 50: Ausleger
- 52: innerer Teleskopmastabschnitt
- 54: Rotor
- 56: Leuchtmittel
- 58: Sensor
- 60: Rotorebene
- 62: Ebene der Leuchtmittel-Anordnung
- 64: Endplatte
- 66: Stab
- 68: Transportband

## Patentansprüche

1. Vorrichtung (10) zur Desinfektion eines Innenraums mit
• einem Wagen, der dazu eingerichtet ist, entlang eines Wegs durch den Innenraum zu fahren, und
• einer germiziden Lichtquelle (36), **gekennzeichnet durch**
• eine Drohne (24), die zur Erzeugung eines Auftriebs mindestens einen elektrischen Antrieb aufweist, wobei
• die germizide Lichtquelle (36) an der Drohne (24) befestigt ist und
• die Drohne (24) zur Energieversorgung der germiziden Lichtquelle (36) und des elektrischen Antriebs über eine elektrische Leitung (22) mit dem Wagen verbunden ist.

2. Vorrichtung (10) nach Anspruch 1, **dadurch gekennzeichnet, dass** der Innenraum eine Flugzeugkabine und der Weg ein Gang der Flugzeugkabine ist.

3. Vorrichtung (10) nach Anspruch 1 oder 2, **dadurch gekennzeichnet, dass** die germizide Lichtquelle (36) eine UVC-Lichtquelle ist, insbesondere mit maximaler Intensität im Wellenlängenbereich von 240 nm bis 100 nm.

4. Vorrichtung (10) nach einem der Ansprüche 1 bis 3, **dadurch gekennzeichnet, dass** die UVC-Lichtquelle eine Exzimer-Lampe ist, insbesondere eine Krypton-Chlorid-Exzimer-Lampe.

5. Vorrichtung (10) nach einem der Ansprüche 1 bis 4, **dadurch gekennzeichnet, dass** die germizide Lichtquelle (36) zwei röhrenförmige Leuchtmittel (56) aufweist, die parallel nebeneinander angeordnet sind.

6. Vorrichtung (10) nach Anspruch 5, **dadurch gekennzeichnet, dass** an zwei gegenüberliegenden Enden der Anordnung der Leuchtmittel (56) jeweils zwei Rotoren (54) der Drohne (24) angeordnet sind.

7. Vorrichtung (10) nach Anspruch 5 oder 6, **dadurch gekennzeichnet, dass** eine von den beiden parallel angeordneten, röhrenförmigen Leuchtmitteln (56) definierte Ebene (62) in einem Winkel im Bereich von 5° bis 45° relativ zu einer Rotorebene (60 geneigt angeordnet ist.

8. Vorrichtung (10) nach einem der Ansprüche 1 bis 7, **dadurch gekennzeichnet, dass** in dem Innenraum mindestens eine Sitzreihe (46) angeordnet ist und die Drohne (24) eine elektronische Steuerung aufweist, die dazu eingerichtet ist, die Drohne (24) automatisch so zu steuern, dass die Drohne (24) sich mit einer vorgegebenen Fluggeschwindigkeit entlang der mindestens einen Sitzreihe (46) bewegt.

9. Vorrichtung (10) nach einem der Ansprüche 1 bis 8, **dadurch gekennzeichnet, dass** der Wagen einen ausfahrbaren Mast (20) aufweist, der in einer eingefahrenen Position im Inneren des Wagens aufgenommen ist und in einer ausgefahrenen Position nach oben aus dem Wagen herausragt, wobei die elektrische Leitung (22) mit dem Mast (20) verbunden ist.

10. Vorrichtung (10) nach einem der Ansprüche 1 bis 9, **dadurch gekennzeichnet, dass** die Vorrichtung (10) einen elektrischen Fahrantrieb und eine elektronische Steuerung für den Wagen aufweist, wobei die Steuerung dazu eingerichtet ist, den Wagen autonom entlang des Wegs zu steuern.

11. Vorrichtung (10) nach einem der Ansprüche 1 bis 10, **dadurch gekennzeichnet, dass** die Vorrichtung (10) ein Stromkabel (30) umfasst, das mit dem Wagen verbunden ist und einen Stecker zum Anschließen an ein Versorgungsnetz, insbesondere ein Flugzeugbordnetz, aufweist.

12. Vorrichtung (10) nach Anspruch 11, **dadurch gekennzeichnet, dass** die Vorrichtung (10) eine Aufrollvorrichtung (28) für das Stromkabel (30) aufweist, die dazu ausgebildet ist, den Wagen durch Aufrollen des Stromkabels (30) entlang eines vorgegebenen Fahrwegs zu ziehen.

13. Vorrichtung (10) nach Anspruch 11 oder 12, **dadurch gekennzeichnet, dass** die Vorrichtung (10) eine Aufrollvorrichtung (10) für das Stromkabel (30) aufweist, wobei die elektronische Steuerung für den Wagen dazu ausgebildet ist, das Stromkabel (30) während der Fahrt automatisch in Abhängigkeit von einer Fahrgeschwindigkeit und/oder Fahrtrichtung auf- und abzurollen.

14. Vorrichtung (10) nach einem der Ansprüche 1 bis 13, **dadurch gekennzeichnet, dass** die Vorrichtung (10) ein Fahrantriebsmodul (18) aufweist, auf dem der Wagen angeordnet und befestigt werden kann, wobei das Fahrantriebsmodul (18) insbesondere ein Raupenfahrwerk aufweist.

15. Vorrichtung (10) nach einem der Ansprüche 1 bis 14, **dadurch gekennzeichnet, dass** der Wagen ein Aufnahmefach (26) für die Drohne (24) und/oder ein Aufnahmefach (16) für das Fahrantriebsmodul (18) aufweist, so dass die Drohne (24) einschließlich der elektrischen Versorgungsleitung (22) bzw. das Fahrantriebsmodul (18) im Inneren des Wagens verstaut werden kann.

## Claims

1. A device (10) for disinfecting an interior space comprising
• a cart, which is configured to travel along a path through the interior space, and
• a germicidal light source (36), **characterized by**
• a drone (24), which has at least one electric drive for generating lift, wherein
• the germicidal light source (36) is fastened to the drone (24) and
• the drone (24) is connected to the cart via an electrical line (22) for supplying energy to the germicidal light source (36) and the electric drive.

2. The device (10) according to claim 1, **characterized in that** the interior space is an aircraft cabin and the path is an aisle of the aircraft cabin.

3. The device (10) according to claim 1 or 2, **characterized in that** the germicidal light source (36) is a UVC light source, in particular with a maximum intensity in the wavelength range from 240 nm to 100 nm.

4. The device (10) according to one of claims 1 to 3, **characterized in that** the UVC light source is an excimer lamp, in particular a krypton chloride excimer lamp.

5. The device (10) according to one of claims 1 to 4, **characterized in that** the germicidal light source (36) has two tubular illuminants (56) arranged parallel next to each other.

6. The device (10) according to claim 5, **characterized in that** two rotors (54) of the drone (24) are arranged on each of two opposing ends of the arrangement of the illuminants (56).

7. The device (10) according to claim 5 or 6, **characterized in that** a plane (62) defined by the two parallel, tubular illuminants (56) is arranged tilted at an angle in the range of 5° to 45° relative to a rotor plane (60).

8. The device (10) according to one of claims 1 to 7, **characterized in that** at least one row of seats (46) is arranged in the interior space and the drone (24) has an electronic control, which is configured to control the drone (24) automatically such that the drone (24) moves at a specified flight speed along the at least one row of seats (46).

9. The device (10) according to one of claims 1 to 8, **characterized in that** the cart has an extendable mast (20), which in a retracted position is accommodated inside the cart and in an extended position projects upwards out of the cart, wherein the electrical line (22) is connected to the mast (20).

10. The device (10) according to one of claims 1 to 9, **characterized in that** the device (10) has an electric travel drive and an electronic control for the cart, wherein the control is configured to control the cart autonomously along the path.

11. The device (10) according to one of claims 1 to 10, **characterized in that** the device (10) comprises a power cable (30) that is connected to the cart and has a plug for connecting to a supply network, in particular an on-board electrical system of an aircraft.

12. The device (10) according to claim 11, **characterized in that** the device (10) has a coiling device (28) for the power cable (30), which is designed to pull the cart along a specified travel path by coiling the power cable (30).

13. The device (10) according to claim 11 or 12, **characterized in that** the device (10) has a coiling device (10) for the power cable (30), wherein the electronic control for the cart is designed to coil and uncoil the power cable (30) automatically during travel depending on a travel speed and/or travel direction.

14. The device (10) according to one of claims 1 to 13, **characterized in that** the device (10) has a travel drive module (18), on which the cart can be arranged and fastened, wherein the travel drive module (18) has in particular a crawler track.

15. The device (10) according to one of claims 1 to 14, **characterized in that** the cart has an accommodation compartment (26) for the drone (24) and/or an accommodation compartment (16) for the travel drive module (18), so that the drone (24) including the electrical supply line (22) or, respectively, the travel drive module (18) can be stowed inside the cart.

## Revendications

1. Dispositif (10) pour la désinfection d'un espace intérieur avec
• un chariot, lequel est agencé pour se déplacer le long d'un chemin à travers l'espace intérieur, et
• une source lumineuse germicide (36), **caractérisé par**
• un drone (24), lequel présente au moins un entraînement électrique pour la génération d'une portance,
• la source lumineuse germicide (36) étant fixée sur le drone (24) et
• le drone (24) étant raccordé au chariot par une ligne électrique (22) pour l'alimentation en énergie de la source lumineuse germicide (36) et de l'entraînement électrique.

2. Dispositif (10) selon la revendication 1, **caractérisé en ce que** l'espace intérieur est une cabine d'aéronef et le chemin un couloir de la cabine d'aéronef.

3. Dispositif (10) selon la revendication 1 ou 2, **caractérisé en ce que** la source lumineuse germicide (36) est une source lumineuse UVC, en particulier avec intensité maximum dans la plage de longueur d'onde de 240 nm à 100 nm.

4. Dispositif (10) selon l'une des revendications 1 à 3, **caractérisé en ce que** la source lumineuse UVC est une lampe excimère, en particulier une lampe excimère krypton-chlorure.

5. Dispositif (10) selon l'une des revendications 1 à 4, **caractérisé en ce que** la source lumineuse germicide (36) comprend deux moyens d'éclairage (56) tubulaires, lesquels sont disposés parallèlement l'un à l'autre.

6. Dispositif (10) selon la revendication 5, **caractérisé en ce que** deux rotors (54) du drone (24) sont respectivement disposés à deux extrémités opposées de l'agencement des moyens d'éclairage (56).

7. Dispositif (10) selon la revendication 5 ou 6, **caractérisé en ce qu'**un plan (62) défini par les deux moyens d'éclairage (56) tubulaires disposés parallèlement est disposé avec un angle dans la plage de 5° à 45° par rapport à un plan de rotor (60).

8. Dispositif (10) selon l'une des revendications 1 à 7, **caractérisé en ce qu'**au moins une série de sièges (46) est disposée dans l'espace intérieur et **en ce que** le drone (24) présente une commande électronique, laquelle est agencée pour piloter automatiquement le drone (24) de telle sorte que le drone (24) se déplace avec une vitesse de vol spécifiée le long de l'au moins une rangée de sièges (46).

9. Dispositif (10) selon l'une des revendications 1 à 8, **caractérisé en ce que** le chariot présente un mât déployable (20), lequel est accueilli à l'intérieur du chariot dans une position rentrée et dépasse hors du chariot vers le haut dans une position sortie, la ligne électrique (22) étant raccordée au mât (20).

10. Dispositif (10) selon l'une des revendications 1 à 9, **caractérisé en ce que** le dispositif (10) présente un système d'entraînement électrique et une commande électronique pour le chariot, la commande étant agencée pour piloter le chariot de façon autonome le long du chemin.

11. Dispositif (10) selon l'une des revendications 1 à 10, **caractérisé en ce que** le dispositif (10) comprend un câble électrique (30), lequel est raccordé au chariot et présente une prise pour le raccordement à un réseau d'alimentation, en particulier un réseau de bord d'aéronef.

12. Dispositif (10) selon la revendication 11, **caractérisé en ce que** le dispositif (10) présente un dispositif d'enroulement (28) pour le câble électrique (30), lequel est conçu pour tracter le chariot par enroulement du câble électrique (30) le long d'un trajet spécifié.

13. Dispositif (10) selon la revendication 11 ou 12, **caractérisé en ce que** le dispositif (10) présente un dispositif d'enroulement (10) pour le câble électrique (30), la commande électronique pour le chariot étant conçue pour enrouler et dérouler automatiquement le câble électrique (30) pendant le déplacement, en fonction d'une vitesse de déplacement et/ou d'une direction de déplacement.

14. Dispositif (10) selon l'une des revendications 1 à 13, **caractérisé en ce que** le dispositif (10) présente un module de système d'entraînement (18), sur lequel le chariot peut être disposé et fixé, le module de système d'entraînement (18) présentant en particulier un train de roulement à chenilles.

15. Dispositif (10) selon l'une des revendications 1 à 14, **caractérisé en ce que** le chariot présente un compartiment d'accueil (26) pour le drone (24) et/ou un compartiment d'accueil (16) pour le module de système d'entraînement (18), de telle sorte que le drone (24) y compris la ligne d'alimentation électrique (22) ou le module de système d'entraînement (18) peuvent être rangés à l'intérieur du chariot.
